# EUROPEAN PATENT APPLICATION

(11) **EP 1 408 028 A1**
(43) Date of publication of application: **14.04.2004**
(21) Application number: 02292494.8
(22) Date of filing: 09.10.2002
(51) Int. Cl.: C07C 281/18, A61K 31/15

(54) **S-adenosyl methionine decarboxylase inhibition for the treatment of a herpes simplex virus infection**

(71) Applicant: INSTITUT NATIONAL DE LA SANTE ET DE LA RECHERCHE MEDICALE (INSERM), 75013 Paris (FR)
(72) Inventor: Diaz, Jean-Jacques, 69190 Saint Fons (FR); Greco, Anna, 69004 Lyon (FR)
(74) Representative: Martin, Jean-Jacques

(57) **Abstract**

The invention relates to the inhibition of S-adenosyl methionine decarboxylase for the prevention and/or the treatment of a herpes simplex virus infection.

## Description

The invention relates to the use of an inhibitor of S-adenosyl methionine decarboxylase for the manufacture of a drug intended for the prevention and/or the treatment of a herpes simplex virus infection.

The object of the invention is to provide a new pathway to inhibit the replication of *herpes simplex* virus, *e.g.* type 1 (HSV-1) or type 2 (HSV-2) in a host cell. These viruses are world-wide distributed, and they are transmitted from infected to susceptible individuals during close personal contact. Herpes infections are very frequent and are generally localized in the face and in the trunk in the case of HSV-1, and in the genital sphere in the case of HSV-2. Currently, the prevalence of *herpes simplex* infection is between 70 and 90 % of the world's adult population. After the primary infection which can be asymptotic, these viruses persist in an apparently inactive state, the latent state. It is estimated that over one third of the world's population have recurrent HSV infections between 3 and 5 times a year, and therefore, the capability of transmitting HSV during episodes of productive infection. The viral reactivation occurs generally after a fever, a stress, an exposure to ultraviolet light, a menstrual cycle, pregnancy, etc. The rythm and the intensity of the reactivation vary from a person to another. The clinical manifestations of the infection and of the reactivation are numerous. Some of them are very severe and can lead to the death of the patients. A study reveals that 61 % of the children, less than one year old, contaminated at the time of delivery and who present a spread HSV infection, die as a consequence of this infection. In addition, patients compromised by immunosuppressive therapy (drug therapy treatment, bone marrow or organ transplantation), or after infection by HIV, develop severe HSV infections with extensive deterioration of the patients. Moreover, herpes infections are the most common causes of genital ulcers in industrialized countries. The prevalence of genital herpes is between 20 % to 90 % of the world's adult population according to the sociocultural context. Most of them are due to HSV-2. However, today one can note that an increasing proportion is caused by HSV-1 (this proportion is 30 % or more). The mostly severely affected population is the new-born children who are infected at the time of the birth and who present irreversible neurological damages in spite of anti-viral therapy. This population is going to increase because of the constant increase of genital herpes infections during the last decades.

For more than 15 years, acyclovir has been accepted as the treatment of choice for the management of HSV infections. Acyclovir is a nucleoside analogue which specifically inhibits the activity of two viral enzymes, the DNA polymerase and the thymidine kinase. However, as with other viruses, the distribution of variants of HSV-1 can shift in response to drug therapy. Indeed, the clinical use of acyclovir is associated with the emergence of drug-resistant virus, isolated from acyclovir treated patients as a result of mutations in the DNA sequences coding for the viral DNA polymerase and thymidine kinase. The prevalence is 6% of the immunocompromised patients. At present, all available anti-herpetic drugs act on these viral enzymes and thus increase the risks of developing resistant virus strains. This is also the case for new drugs announced to be in development, that inhibit another viral enzyme, the helicase primase.

The invention proposes an innovative approach to avoid the emergence of drug-resistant virus. This alternative consists in the development of molecules with anti-viral effect directed against cellular proteins which intervene in the life cycle of the virus instead of viral proteins.

It is well established that HSV-1 protein synthesis is concomitant to a repression of host protein synthesis which is selective and progressive and occurs very early after infection. However, whereas the synthesis of most host proteins is shut off, a small number of cellular proteins continues to be efficiently synthesized upon infection. This is the case for all the ribosomal proteins and for some of non ribosomal proteins (Greco et *al*., 2000; Greco et al., 1997).

In particular, one of these proteins has been identified by the inventors as involved in the polyamines biosynthetic pathway. The end products of this pathway are spermine and spermidine. These two products are found in different substructures of the mature virion : spermine is restricted to nucleocapsid, and spermidine to the viral envelope (Gibson and Roizman, 1971). Accordingly, the inventors tested whether enzymes involved in the metabolic pathway of polyamines escape the virally-induced shutoff of cellular protein synthesis.

Actually, the synthesis of ornithine decarboxylase (ODC), which decarboxylates ornithine into putrescine, was found to be stimulated upon infection. Therefore, inhibition of ODC as well as other key enzymes of polyamines synthesis was tested for prevention of viral replication.

Various groups have published contradictory results on the relation of ODC activity and HSV-1 replication. Several authors demonstrated either that difluoromethylornithine (DFMO) which inactivates ODC, or that methylglyoxal bis(guanylhydrazone) (MGBG) which specifically inhibits the activity of S adenosyl methionine decarboxylase (SAMDC), have no effect on HSV replication (McCormick and Newton, 1975; Tyms *et al*., 1979). On the contrary, others showed that DFMO have an inhibitory effect on HSV-1 infection when used at millimolar dose (Pohjanpelto *et al.,* 1988). ODC decarboxylates ornithine into putrescine, whereas SAMDC decarboxylates S-adenosyl methionine (SAM) into decarboxylated SAM (dcSAM) which is used as a substrate in both synthesis of spermidine from putrescine, and of spermine from spermidine.

Since that time, no further study has been published on this subject matter. The inventors now demonstrate for the first time that blocking SAMDC activity makes it possible to achieve efficient inhibition of HSV replication. More particularly, the results presented herein show that MGBG inhibits HSV-1 replication *in vitro* at concentrations which are not toxic for the cells.

In addition, MGBG reveals to inhibit *in vitro* the replication of viral mutants HSV-1 strains that are resistant to conventional antiviral drugs, e.g. acyclovir and foscarnet. Actually, as with other viruses, the distribution of variants of HSV-1 can shift in response to drug therapy. Although HSV-1 virus replication is often controllable using nucleotide analogues which inhibit the viral DNA polymerase or thymidine kinase, drug-resistant variants often emerge following chronic treatment, as a result of mutations in the DNA sequence coding for these enzymes. Because the infections by HSV are in outbreak, and because drugs which are available at present are directed against a viral protein, appearance of viral mutants that become resistant to acyclovir and its byproducts has become a major problem.

Accordingly, the development of an antiviral strategy targeting a cellular component necessary for the viral replication, such as S adenosyl methionine decarboxylase, rather than a viral component makes it possible to avoid emergence of drug-resistant viruses.

### Definitions

"S adenosyl methionine decarboxylase" or "SAMDC" denotes the enzyme that decarboxylates S-adenosyl methionine (SAM) into decarboxylated SAM (dcSAM). In particular, human SAMDC coding sequence (SEQ ID N°1) is deposited in Genebank, under accession number M21154.

A "*S-adenosyl methionine decarboxylase inhibitor*" or "*SAMDC inhibitor*" is defined herein as a compound which: (i) inhibits the activity and/or expression of SAMDC *in vitro* and/or *in vivo*; and/or (ii) blocks decarboxylation of S-adenosyl methionine (SAM) into decarboxylated SAM (dcSAM); and/or (iii) blocks intracellular synthesis of spermidine from putrescine, and of spermine from spermidine. Inhibition and blocking may be total or partial.

As used herein, the term *"herpes simplex* virus" (HSV) is intended for viruses from the Alphaherpesvirinae subfamily of herpes viruses that belong to the genus simplexvirus. Examples of HSV include the human *herpes simplex* virus type 1 (HSV-1) or type 2 (HSV-2), the bovine *herpes* virus 2 (BoHV-2), or the *herpes* virus B (HBV) also called *Herpesvirus simiae.* Preferred *herpes simplex* viruses are HSV-1 or HSV-2, and in particular HSV-1 or HSV-2 strain resistant to an agent efficient against a herpes simplex virus infection.

In the context of the invention, the term "*agent efficient against a herpes simplex virus infection*" is meant for a conventional antiviral drug that is directed against a viral protein rather than a cellular target. Examples of such a reference treatment include for instance nucleoside analogues which inhibit the viral DNA polymerase or thymidine kinase. Typical examples of conventional antiviral drugs are acyclovir, foscarnet, valacyclovir, gancyclovir, famcyclovir, pencyclovir, cidofovir, idoxuridine, trifluridine, vidarabine, and derivatives thereof. Such conventional antiviral chemotherapeutic agents are reviewed in Harrison (2000). Agents efficient against a herpes simplex virus infection also include non nucleoside-analogue compounds, such as thiazoyl-phenyl-containing inhibitors, that target HSV helicase-primase enzyme.

The term "*herpes simplex virus infection*" denotes the condition of a subject infected with HSV, either in a primary or a latent infectious state, as well as in a reactivation period. Primary infection generally occurs through a break in the mucus membranes of the mouth or throat, via the eye or genitals or directly via minor abrasions in the skin. Initial infection is usually asymptomatic, although there may be minor local vesicular lesions. Local multiplication ensues, followed by viraemia and systemic infection. There then follows life-long latent infection with periodic reactivation. The delicate balance of latency may be upset by various disturbances, physical (injury, U.V, hormones, etc) or psychological (stress, emotional upset). Reactivation of latent virus leads to recurrent disease wherein virus travels back to surface of body and replicates, causing tissue damage. HSV-1 is primarily associated with oral and ocular lesions whereas HSV-2 is primarily associated with genital and anal lesions.

Accordingly, *"prevention or treatment of a herpes simplex virus infection"* is meant for the prevention of the onset of a primary infection and/or for the prevention of occurrence of a viral reactivation period in the subject with latent infection, as well as the treatment of the lesions caused by the virus reactivation.

As used herein "*pharmaceutically acceptable carrier*" includes any solvents, dispersion media, coatings, antibacterial and antifungal agents, isotonic and absorption delaying agents and the like that do not produce an adverse, allergic or other untoward reaction when administered to an animal, or a human, as appropriate. The use of such media and agents for pharmaceutical active substances is well known in the art. Except insofar as any conventional media or agent is incompatible with the active ingredient, its use in the therapeutic compositions is contemplated.

### Description of the invention

The invention thus relates to the use of an inhibitor of S-adenosyl methionine decarboxylase (SAMDC) for the manufacture of a medicament intended for the prevention or the treatment of a herpes simplex virus infection.

An embodiment of the invention is the use of an inhibitor of S-adenosyl methionine decarboxylase for the manufacture of a medicament intended for the treatment of a subject to prevent replication of a herpes simplex virus.

The invention further provides a method of treatment of a herpes simplex virus infection comprising administration of an inhibitor of S-adenosyl methionine decarboxylase to a subject in need thereof.

### Inhibition of SAMDC activity

According to an embodiment, said SAMDC inhibitor is a direct inhibitor of SAM DC activity,

Such an inhibitor may be any peptide, peptidomimetics or non peptidic mimetics (Rubin-Carrez, 2000), such as small organic molecules capable of interfering with the enzymatic activity of SAMDC, e.g. through blocking decarboxylation of S-adenosyl methionine (SAM) into decarboxylated SAM (dcSAM); and/or blocking intracellular synthesis of spermidine from putrescine, and of spermine from spermidine.

Inhibitors can be readily identified by screening methods, including biochemical and cellular *in vitro* assays. Of particular interest is a process for screening substances for their ability to inhibit SAMDC activity, comprising the steps of providing a cell that expresses SAMDC and testing the ability of the candidate substances to inhibit, i.e. to block or decrease intracellular synthesis of spermidine from putrescine, and/or of spermine from spermidine. The decrease in the level of spermidine and/or spermine synthesis in comparison with a SAMDC expressing cell that was not subjected to this candidate substance, is indicative of a substance that shows inhibiting activity toward SAM DC.

Alternatively, a process for screening substances for their ability to inhibit SAMDC activity, may comprise the steps of providing SAMDC and testing the ability of the candidate substances to inhibit, i.e. to block or decrease SAM decarboxylation into dcSAM. The decrease in the level of DcSAM synthesis, or a decrease in SAM processing, in comparison with SAMDC not subjected to this candidate substance, is indicative of a substance that shows inhibiting activity toward SAMDC.

Methylglyoxal bis(amidinohydrazone) (MGBG) or derivatives thereof are examples of organic molecule that inhibits SAMDC activity. Thus, preferably said SAMDC inhibitor is methylglyoxal bis(amidinohydrazone) or a derivative thereof provided it retains the capacity to (i) inhibit the activity and/or expression of SAMDC *in vitro* and/or *in vivo;* and/or (ii) inhibit decarboxylation of S-adenosyl methionine (SAM) into decarboxylated SAM (dcSAM); and/or (iii) inhibit intracellular synthesis of spermidine from putrescine, and of spermine from spermidine.

The SAMDC inhibitor may also be a monoclonal or polyclonal antibody, or a fragment thereof, or a chimeric or immunoconjugate antiboby, which is capable of specifically interacting with SAMDC and inhibiting its activity. Alternatively, said inhibitor may be capable of interacting with SAMDC substrate, *i.e.* S-adenosyl methionine (SAM), thereby blocking SAM processing into decarboxylated SAM (dcSAM). The antibodies of the present invention can be single chain or double chain, chimeric antibodies, humanized antibodies, or portions of an immunoglobulin molecule, including those portions known in the art as antigen binding fragments Fab, Fab', F(ab')2 and F(v). They can also be immunoconjugated ,e.g. with a toxine, or labelled antibodies.

Whereas polyclonal antibodies may be used, monoclonal antibodies are preferred for they are more reproducible in the long run.

Procedures for raising polyclonal antibodies are also well known. Polyclonal antibodies can be obtained from serum of an animal immunized against the SAMDC or SAM protein , which can be produced by genetic engineering for example according to standard methods well-known by one skilled in the art. Typically, such antibodies can be raised by administering the protein subcutaneously to New Zealand white rabbits which have first been bled to obtain pre-immune serum. The antigens can be injected at a total volume of 100 µl per site at six different sites. Each injected material will contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed in Harlow et al. (1988) which is hereby incorporated by reference.

A "monoclonal antibody" in its various grammatical forms refers to a population of antibody molecules that contain only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art (see, for example, Harlow et *al*., 1988). Monoclonal antibodies (mAbs) may be prepared by immunizing purified NP protein isolated from any of a variety of mammalian species into a mammal, e.g. a mouse, rat, rabbit, goat, camelides, human and the like mammal. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody. This standard method of hybridoma culture is described in Kohler and Milstein (1975).

While mAbs can be produced by hybridoma culture, the invention is not to be so limited. Also contemplated is the use of mAbs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No. WO 890099 to Robinson *et al.*; European Patent Publications No. 0239400 to Winter et al. and No. 0125023 to Cabilly et *al.*

Antibody generation techniques not involving immunisation are also contemplated such as for example using phage display technology to examine naive libraries (from non-immunised animals); see Barbas *et al.* (1992), and Waterhouse et al. (1993).

Aptamers may also be of interest. Aptamers are a class of molecule that represents an alternative to antibodies in term of molecular recognition. Aptamers are oligonucleotide or oligopeptide sequences with the capacity to recognize virtually any class of target molecules with high affinity and specificity. Oligonucleotidic aptamers may be isolated through Systematic Evolution of Ligands by Exponential enrichment (SELEX) of a random sequence library, as described in Tuerk C. and Gold L. (1990). The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D. (1999). Peptide aptamers consists of a conformationally constrained antibody variable region displayed by a platform protein, such as *E. coli* Thioredoxin A that are selected from combinatorial libraries by two hybrid methods (Colas et *al*., 1996).

### Inhibition of SAMDC expression

According to another embodiment, said SAMDC inhibitor is an indirect inhibitor of SAMDC activity, that decreases its synthesis through inhibition of gene expression. Inhibition of SAMDC expression may be achieved through blocking transcription of SAMDC gene and/ortranslation of SAMDC mRNA.

The invention thus relates to the use of an inhibitor of S-adenosyl methionine decarboxylase (SAMDC) for the manufacture of a medicament intended for the prevention or the treatment of a herpes simplex virus infection, wherein said inhibitor is an inhibitor of SAMDC expression, such as an antisense nucleic acid sequence that blocks expression of SAMDC.

Antisense strategy may be used to interfere with SAMDC expression. This approach may for instance utilize antisense nucleic acids or ribozymes that block translation of a specific mRNA, either by masking that mRNA with an antisense nucleic acid or cleaving it with a ribozyme. For a general discussion of antisense technology, see, e.g., Antisense DNA and RNA, (Cold Spring Harbor Laboratory, D. Melton, ed., 1988).

Reversible short inhibition of SAMDC transcription may also be useful. Such inhibition can be achieved by use of siRNAs. RNA interference (RNAi) technology prevents the expression of genes by using small RNA molecules such as « small interfering RNAs" (siRNAs). This technology in turn takes advantage of the fact that RNAi is a natural biological mechanism for silencing genes in most cells of many living organisms, from plants to insects to mammals (Sharp, 2001). RNAi would prevent a gene from producing a functional protein by ensuring that the molecule intermediate, the messenger RNA copy of the gene is destroyed. siRNAs could be used in a naked form and incorporated in a vector, as described below. One can further make use of aptamers to specifically inhibit SAMDC transcription.

An "antisense nucleic acid" or "antisense oligonucleotide" is a single stranded nucleic acid molecule, which, on hybridizing under cytoplasmic conditions with complementary bases in a RNA or DNA molecule, inhibits the latter's role. If the RNA is a messenger RNA transcript, the antisense nucleic acid is a countertranscript or mRNA-interfering complementary nucleic acid. As presently used, "antisense" broadly includes RNA-RNA interactions, RNA-DNA interactions, ribozymes, RNAi, aptamers and Rnase-H mediated arrest.

Ribozymes are RNA molecules possessing the ability to specifically cleave other single stranded RNA molecules in a manner somewhat analogous to DNA restriction endonucleases. Ribozymes were discovered from the observation that certain mRNAs have the ability to excise their own introns. By modifying the nucleotide sequence of these ribozymes, researchers have been able to engineer molecules that recognize specific nucleotide sequences in an RNA molecule and cleave it (Cech, 1989). Because they are sequence-specific, only mRNAs with particular sequences are inactivated.

Antisense nucleic acid molecules can be encoded by a recombinant gene for expression in a cell (*e.g.*, U.S. patent No 5,814,500; U.S. 5,811,234), or alternatively they can be prepared synthetically (e.g., U.S. patent No 5,780,607).

As used herein, the term "oligonucleotide" refers to a nucleic acid, generally of at least 10, preferably at least 13, and more preferably at least 20 nucleotides, preferably no more than 100 nucleotides, that is hybridizable to a genomic DNA (gDNA) molecule, a complementary DNA (cDNA) molecule, or a messenger RNA (mRNA) molecule encoding a gene, mRNA, cDNA, or other nucleic acid of interest.

Oligonucleotides can be labelled, *e.g.*, with 32P-nucleotides or nucleotides to which a label, such as biotin, has been covalently conjugated. Generally, oligonucleotides are prepared synthetically, preferably on a nucleic acid synthesizer. Accordingly, oligonucleotides can be prepared with non-naturally occurring phosphoester analog bonds, such as thioester bonds, etc.

"SAMDC antisense" nucleic acids may be designed to specifically hybridize with a SAMDC encoding sequence, *e.g.* a sequence form the human SAMDC coding sequence shown in SEQ ID N°1.

A "sequence capable of specifically hybridizing with a nucleic acid sequence" is understood as meaning a sequence which hybridizes with the nucleic acid sequence to which it refers under the conditions of high stringency (Sambrook et al, 1989). These conditions are determined from the melting temperature Tm and the high ionic strength. Preferably, the most advantageous sequences are those which hybridize in the temperature range (Tm - 5°C) to (Tm - 30°C), and more preferably (Tm - 5°C) to (Tm - 10°C). A ionic strength of 6xSSC is more preferred. For instance, high stringency hybridization conditions correspond to the highest Tm, e.g., 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et *al*., 1989). For hybridization with shorter nucleic acids, *i.e.*, oligonucleotides, the position of mismatches becomes more important, and the length of the oligonucleotide determines its specificity (see Sambrook et *al*., 1989).

The antisense nucleic acid sequences according to the invention can be used as such, for example after injection into man or animal, to induce a protection or to treat HSV infection. In particular, they can be injected in the form of naked DNA according to the technique described in application WO 90/11092. They can also be administered in complexed form, for example with DEAE-dextran (Pagano et *al.*, 1967), with nuclear proteins (Kaneda et *al.*, 1989), with lipids (Felgner et *al.*, 1987), in the form of liposomes (Fraley et al., 1980), and the like.

Preferably, the nucleic acid sequences form part of a vector. The use of such a vector indeed makes it possible to improve the administration of the nucleic acid into the cells to be treated, and also to increase its stability in the said cells, which makes it possible to obtain a durable therapeutic effect. Furthermore, it is possible to introduce several nucleic acid sequences into the same vector, which also increases the efficacy of the treatment.

The terms "vector" means the vehicle by which a DNA or RNA sequence (*e.g.* a foreign gene) can be introduced into a host cell, so as to transform the host and promote expression (*e.g.* transcription and translation) of the introduced sequence. Vectors include plasmids, phages, viruses, etc.

The vector used in antisense strategy may be of diverse origin, as long as it is capable of transducing animal cells, and in particular human cells. In a preferred embodiment of the invention, a viral vector is used which can be chosen from adenoviruses, retroviruses, adeno-associated viruses (AAV), lentivirus, herpes virus, cytomegalovirus (CMV), vaccinia virus and the like. Vectors derived from adenoviruses, retroviruses or AAVs, HIV-derived retroviral vectors, incorporating heterologous nucleic acid sequences have been described in the literature (Akli et al., (1993); Stratford-Perricaudet et *al.* (1990) ; EP 185 573, Levrero et *al.* (1991) ; Le Gal la Salle et *al.* (1993) ; Roemer et *al.* (1992) ; Dobson et *al.* (1990) ; Chiocca et al. (1990) ; Miyanohara et *al*. (1992) ; WO 91/18088).

Such vectors generally comprise a promoter sequence, signals for initiation and termination of transcription. Their insertion into the host cell may be transient or stable. These various control signals are selected according to the host cell and may be inserted into vectors which self-replicate in the selected host cell, or into vectors which integrate the genome of said host.

The present invention therefore also relates to any recombinant virus comprising, inserted into its genome, a SAMDC antisense sequence.

Advantageously, the recombinant virus according to the invention is a defective virus. The term "defective virus" designates a virus incapable of replicating in the target cell. Generally, the genome of the defective viruses used within the framework of the present invention is therefore devoid of at least the sequences necessary for the replication of the said virus in the infected cell. These regions can either be removed (completely or partially), or rendered non-functional, or substituted by other sequences and especially by the SAMDC antisense nucleic acid of the invention. Preferably, the defective virus nevertheless conserves the sequences of its genome which are necessary for the encapsulation of the viral particles.

It is particularly advantageous to use the nucleic acid antisense sequences of the invention in a form incorporated in an adenovirus, an AAV or a defective recombinant retrovirus.

As regards adenoviruses, various serotypes exist whose structure and properties vary somewhat, but which are not pathogenic for man, and especially non-immunosuppressed individuals. Moreover, these viruses do not integrate into the genome of the cells which they infect, and can incorporate large fragments of exogenous DNA. Among the various serotypes, the use of the AD5/F35 chimeric adenovirus vector (Yotnda et *al*., 2001) is preferred within the framework of the present invention. In the case of the Ad5 adenoviruses, the sequences necessary for the replication are the E1A and E1B regions.

The defective recombinant viruses of the invention can be prepared by homologous recombination between a defective virus and a plasmid carrying, inter alia, the SAMDC antisense nucleic acid sequence (Levrero et *al*., 1991 ; Graham, 1984). The homologous recombination is produced after co-transfection of the said viruses and plasmid into an appropriate cell line. The cell line used should preferably (i) be transformable by the said elements, and (ii), contain sequences capable of complementing the part of the genome of the defective virus, preferably in integrated form so as to avoid the risks of recombination. As example of a line which can be used for the preparation of defective recombinant adenoviruses, there may be mentioned the human embryonic kidney line 293 (Graham et *al.,* 1977) which contains especially, integrated into its genome, the left part of the genome of an Ad5 adenovirus (12%). As example of a line which can be used for the preparation of defective recombinant retroviruses, there may be mentioned the CRIP line (Danos et *al.*, 1988). Alternative vectors, such as shuttle vectors, can also be used that permit the cloning of the desired gene in the vector backbone.

Then the viruses that have multiplied are recovered and purified according to conventional molecular biology techniques.

Vectors insertion into the host cell may be achieved by transfection or infection.

Antisense oligonucleotides can also be used to provide a transient SAMDC inhibition. For that purpose antisense oligonucleotides, that are not part of a viral vector, can be administered to the cell by any means as described below.

The term "transfection" means the introduction of a foreign nucleic acid into a cell. The term "transformation" means the introduction of a "foreign" (*i.e.* extrinsic or extracellular) gene, DNA or RNA sequence to a host cell, so that the host cell will express the introduced gene or sequence to produce a desired substance, typically an antisense sequence.

The term "host cell" means any cell of any organism that is selected, modified, transformed, grown, or used or manipulated in any way, for the production of a substance by the cell, for example the expression by the cell of a gene, a DNA or RNA sequence, a protein or an enzyme.

Targeted gene delivery is described in International Pat. Publication WO 95/28494, published October 1995.

Alternatively, the antisense nucleic acid sequence, which may be part or not of a vector, can be introduced in vivo by lipofection. For the past decade, there has been increasing use of liposomes for encapsulation and transfection of nucleic acids in vitro. Information regarding liposome is provided in the "pharmaceutical composition" section of the present application as well. Synthetic cationic lipids designed to limit the difficulties and dangers encountered with liposome mediated transfection can be used to prepare liposomes for *in vivo* transfection of a nucleic acid sequence (Felgner et *al*., 1987). The use of cationic lipids may promote encapsulation of negatively charged nucleic acids, and also promote fusion with negatively charged cell membranes (Felgner et *al.*, 1989). The use of lipofection to introduce exogenous genes into the specific organs in vivo has certain practical advantages. Molecular targeting of liposomes to specific cells represents one area of benefit. It is clear that directing transfection to particular cell types would be particularly advantageous in a tissue with cellular heterogeneity, such as pancreas, liver, kidney, and the brain. Lipids may be chemically coupled to other molecules for the purpose of targeting. Targeted peptides, e.g. hormones or neurotransmitters, and proteins such as antibodies, or non-peptide molecules could be coupled to liposomes chemically.

It is also possible to introduce *in vivo* the antisense nucleic acid sequence, which may be part or not of a vector, as a naked DNA plasmid. Naked DNA vectors can be introduced into the desired host cells by methods known in the art, *e.g.*, transfection, electroporation, microinjection, transduction, cell fusion, DEAE dextran, calcium phosphate precipitation, use of a gene gun, or use of a DNA vector transporter (see, *e.g.*, Wilson et *al*., 1992 ; Wu et *al*., 1988).

### Therapeutics

In the context of the present invention, the subject infected with HSV is a mammal, including a human. Examples of non-human mammals include cattle such as cow, sheep, goat, swine, a pet such as dog or cat, or a rodent. As regards to human subjects, the invention may prove particularly useful for the prevention of herpes infection in immunodepressed patients. The immunocompromised condition may have been acquired following chemotherapeutic treatment, grafting and subsequent immunosuppressive treatment or HIV infection. Such patients often develop severe HSV infections with extending lesions that are difficult to control and sometimes evolution toward encephalitis. Furthermore, the occurrence of mutant viral strains resistant to classical anti-HSV drugs is increased in these patients.

The invention thus concerns the use of an inhibitor of S-adenosyl methionine decarboxylase for the manufacture of a medicament for the prevention or the treatment of a herpes simplex virus infection, wherein said medicament is intended for administration to a mammal. More particularly said mammal is a human, preferably a immunodepressed subject.

Another aspect of the invention is the use of an inhibitor of S-adenosyl methionine decarboxylase in association with another agent efficient against a herpes simplex virus infection, for instance acyclovir and/or foscarnet, for the manufacture of a medicament for the prevention or the treatment of a herpes simplex virus infection.

Accordingly, the invention further provides a method of treatment of a herpes simplex virus infection comprising administration of an inhibitor of S-adenosyl methionine decarboxylase and of another agent efficient against a herpes simplex virus infection to a subject in need thereof.

Said inhibitor of S-adenosyl methionine decarboxylase and said agent efficient against a herpes simplex virus infection can be administrated simultaneously or separately in time, formulated in a same pharmaceutical composition or in different compositions.

### Pharmaceutical compositions

The invention thus further provides a pharmaceutical composition comprising an inhibitor of S-adenosyl methionine decarboxylase and another agent efficient against a herpes simplex virus infection, in a pharmaceutically acceptable carrier. Preferably, said agent efficient against a herpes simplex virus infection is selected from the group consisting of acyclovir, foscarnet, valacyclovir, gancyclovir, famcyclovir, pencyclovir, cidofovir, idoxuridine, trifluridine, vidarabine, and derivatives thereof.

Also preferably, said SAMDC inhibitor is methylglyoxal bis(amidinohydrazone) or a derivative thereof.

The pharmaceutical compositions of the invention, including further the active material a convenient vehicle, may be administered to a mammal, preferably to a human, in need of a such treatment, according to a dosage which may vary widely as a function of the age, weight and state of health of the patient, the nature and severity of the complaint and the route of administration. The appropriate unit forms of administration comprise oral forms such as tablets, gelatin capsules, powders, granules and oral suspensions or solutions, sublingual and buccal administration forms, topical, parenteral, subcutaneous, transcutaneous, transungal, intramuscular (e.g. by injection or by electroporation), intravenous, intranasal or intraoccular administration forms and rectal administration forms.

Preferably, the pharmaceutical compositions contain vehicles which are pharmaceutically acceptable for a formulation capable of being administered subcutaneously.

The suitable pharmaceutical compositions may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

To prepare pharmaceutical compositions for peptide or antibody therapy, an effective amount of the protein may be dissolved or dispersed in a pharmaceutically acceptable carrier or aqueous medium.

Examples of pharmaceutical formulations are provided hereafter.

Pharmaceutical compositions comprise an effective amount of a SAMDC inhibitor in a pharmaceutically acceptable carrier or aqueous medium.

"Pharmaceutically" or "pharmaceutically acceptable" refer to molecular entities and compositions that do not produce an adverse, allergic or other untoward reaction when administered to an animal, or a human, as appropriate.

As used herein, a "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The pharmaceutical forms suitable for injectable use include sterile aqueous solutions or dispersions ; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In terms of using peptide therapeutics as active ingredients, U.S. patents 4,608,251 ; 4,601,903 ; 4,599,231 ; 4,599,230 ; 4,596,792 and 4,572,770 provide useful information.

The preparation of more, or highly concentrated solutions for direct injection is also contemplated, where the use of DMSO as solvent is envisioned to result in extremely rapid penetration thereby delivering high concentrations.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The SAMDC inhibitors may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, *e.g.* tablets or other solids for oral administration ; liposomal formulations ; time release capsules ; and any other form currently used, including creams.

Other routes of administration are contemplated, including nasal solutions or sprays, aerosols or inhalants, or vaginal or rectal suppositories and pessaries.

In certain embodiments, the use of liposomes and/or nanoparticles is contemplated for the introduction of inhibitory antibodies or other agents, especially protein or peptide agents, as well as nucleic acid vectors into host cells. The formation and use of liposomes is generally known to those of skill in the art, and is also described below.

Nanocapsules can generally entrap compounds in a stable and reproducible way. To avoid side effects due to intracellular polymeric overloading, such ultrafine particles (sized around 0.1 µm) should be designed using polymers able to be degraded in vivo. Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core.

The following information may also be utilized in generating liposomal formulations. Phospholipids can form a variety of structures other than liposomes when dispersed in water, depending on the molar ratio of lipid to water. At low ratios the liposome is the preferred structure. The physical characteristics of liposomes depend on pH, ionic strength and the presence of divalent cations. Liposomes can show low permeability to ionic and polar substances, but at elevated temperatures undergo a phase transition which markedly alters their permeability. The phase transition involves a change from a closely packed, ordered structure, known as the gel state, to a loosely packed, less-ordered structure, known as the fluid state. This occurs as a characteristic phase-transition temperature and results in an increase in permeability to ions, sugars and drugs.

Liposomes interact with cells via four different mechanisms: endocytosis by phagocytic cells of the reticuloendothelial system such as macrophages and neutrophils ; adsorption to the cell surface, either by non-specific weak hydrophobic or electrostatic forces, or by specific interactions with cell-surface components ; fusion with the plasma cell membrane by insertion of the lipid bilayer of the liposome into the plasma membrane, with simultaneous release of liposomal contents into the cytoplasm ; and by transfer of liposomal lipids to cellular or subcellular membrane, or vice versa, without any association of the liposome contents. Varying the liposome formulation can alter which mechanism is operative, although more than one may operate at the same time.

The pharmaceutical compositions of the invention are useful for preventing or treating HSV infections.

The invention will be further understood in view of the following examples and the annexed figures.

### Figures

**Figure 1** is a schematic representation of the metabolic pathway of polyamines.

**Figure 2** displays the percent of syncytia present in HEp2 cells infected with 0,5 PFU of HSV-1 per cell and treated with 10-300 µM MGBG as compared to that of untreated infected cells. HEp2 cells were either pre-treated with MGBG for 6 or 24 hours prior infection, or not.

### Examples

### EXAMPLE 1 : Materiel and methods

### Cell lines and virus strain.

HEp2 cells were grown as monolayers in Eagle's minimum essential medium (E-MEM) supplemented or not with 5% heat-inactivated foetal calf serum (FCS) and supplemented with 100 U/ml penicillin and 100 U/ml streptomycin. The cells were maintained at 37°C under 5% CO₂. The HSV-1 macroplaque strain (MP), obtained from B. Jacquemont (Lyon, France), was a gift of B. Roizman (Chicago, III., U.S.A.), and was used throughout this study. Viruses were grown in HEp2 cells.

### Infection of cells and MGBG treatment.

HEp2 cells were grown in 6-well plates (∼10⁶ cells/well) and infected just before confluence with a multiplicity of infection (m.o.i.) of 0.5 plaque-forming units (PFU) per cell in E-MEM in absence of FCS, except for some experiments. After adsorption of the virus for 1 h at 33°C under 5% CO₂, the medium containing the virus suspension was removed and the cells were washed with E-MEM, then incubated in E-MEM without FCS at 37°C for different periods of times until harvest. Times post-infection (p.i.) were calculated from the time of addition of the virus suspension. HEp2 cells were exposed to MGBG treatment for different times. When cells were pre-treated with MGBG prior infection, MGBG was added to cell medium 6 h or 24 h prior infection until harvest. In non pre-treated cells, MGBG was added simultaneously with virus suspension, or 2 h, 4 h or 6 h latter. When cells were treated for more than 24 h, medium was replaced every 24 h with fresh medium containing the same concentration of MGBG.

### Estimation of the viability of HEp2 cells.

Uninfected HEp2 cells were grown in the absence (control) and the presence of 10 to 500 µM of MGBG for 24 h, 30 h and 48 h. Every 24 h, medium was replaced with fresh medium containing the same concentration of MGBG. At the time of harvesting, culture medium was collected from wells of untreated control cells and treated cells. Then cells from the monolayers were gently trypsinized and added to the corresponding previous cell culture medium. Total cells were collected by gentle centrifugation at 500xg and were resuspended in PBS containing 0.2% trypan blue. For each experiment, total cells, living cells and dead cells were counted.

### Spermine quantitation.

HEp2 cells were grown in 6-well plates (∼10⁶ cells/well). Cells were infected or treated with 100 µM MGBG for 12 h or 24 h. For HSV-1-infected cells, MGBG was added simultaneously with viral suspension. Cell extracts and standards (spermine and diaminoheptane as an internal standard, (DAH)) were dansylated according to the procedure described by Seiler (Seiler, 1970) and were then treated using the protocol adapted from Besson (Besson *et al.,* 1986). Dansylation was proceeded in glass vials by mixing 40 µl of HClO₄ extracts, 10 µl of DAH , and 100 µl of 0.3 M Na₂CO₃ (Merck). The reaction was initiated by adding 200 µl of freshly prepared dansyl chloride solution (5mg/ml) in acetone (SDS, spectrosol grade) and allowed to proceed overnight in the dark at room temperature. After dansylation, each sample was diluted with 700 µl H₂O, vortexed and applied to a Waters Sep-Pak C18 cartridge (Birnbaum *et al.,* 1988). After washing with 4 ml of 20 % methanol, the polyamine containing fraction was eluted with 2 ml of 100 % methanol. The separation of polyamines and the quantification of spermine were performed by reverse phase high performance chromatography (RP-HPLC) using a Waters system composed of two models 510 pumps, a Wisp 700 autosampler and a NEC APC4 data module recorder integrator. A Merck F 1050 fluorescence spectrophotometer detected fluorescence (350 nm excitation and 495 nm emission). The separations were performed on a RP18 Merck Lichrocart (25 X 4 mm, 5 µm) precolumn and a RP18, 100 CH Merck column (125 X 4 mm; spherical packing 5 µm). The solvent system was an acetonitrile/water gradient at a flow rate of 1 ml/min of acetonitrile 60 % in water for 7 min, then acetonitrile 90 % for 10 min and a 98 % acetonitrile purge for 5 min. The column was reequilibrated to initial 60 % acetonitrile conditions during 10 min between two successive injections. For each determination, 50 or 100 µl of dansylated samples were injected onto the column equilibrated in 60 % acetonitrile. Spermine was identified by its retention time compared to that of standard spermine. Peak areas were automatically measured by the integrator and evaluated according to the calibration method (Birnbaum *et al.,* 1988). Spermine standard from 10 to 70 pmol was reacted and chromatographed to establish linear standard curve which was used to estimate the absolute amounts of spermine. The absolute limit of detection per injection was 1 pmol for dansylated spermine. Two blank injections were routinely run between calibrations and sample analysis. Mock-infected cells grown in the absence of MGBG were used as the control of intracellular spermine content and was therefore used as 100 % value.

### Measurement of the amount of mRNAs and DNA.

Total RNA and DNA from control mock-infected and infected HEp2 cells were purified using Qiagen RNA/DNA kit. RNA were submitted to DNAse/RNAse free digestion (1.5 unit/µg RNA) and DNA were submitted to RNAse A digestion (10 units/µg). Quantitation of SAMDC mRNA, of viral mRNAs and viral DNA present in each sample was determined by slot blot analysis using the 1250 bp Pstl/Pvull fragment of pSAMr1 plasmid (Pajunen et al., 1988). The 746 bp Sal*I*-Eco*NI* fragment of pSG28 and the 1308 bp Mlu/ fragment of pSG124 plasmids (Goldin *et al.,* 1981) were labelled with [³²P] and used as probes to detect the immediate early ICP27 and the early UL42 mRNAs respectively. Us11 late mRNA was detected with [³²P]-labelled probe composed of the 230 bp Xhol fragment of pHSV-Us11 plasmid (Diaz *et al.,* 1996). Viral DNA was detected using the ICP27 specific DNA probe. Hybridized viral mRNAs and DNA were revealed and quantified by scanning densitometry of the membranes with a Phosphorlmager SI (APB).

### Detection of viral proteins.

Mock-infected and HSV-1-infected HEp2 cells were washed three times with ice cold phosphate-buffered saline (PBS) (130 mM NaCl, 4 mM Na₂HPO₄.2H₂O, 1.5 mM KH₂PO₄), scrapped into PBS, and collected by centrifugation at 500xg. Cells were resuspended in Laemmli buffer (Laemmli, 1970). Proteins were resolved by polyacrylamide gel electrophoresis and transferred by electroblotting onto a polyvinylidene difluoride membrane (Immobilon-P; Millipore). Viral proteins were analysed by Western blots (10 µg aliquots) using a 50 fold dilution of either a rabbit polyclonal anti-ICP27 antibody, or a rabbit polyclonal anti-Us11 antibody (Diaz *et al.,* 1993), or a mouse monoclonal anti-UL42 antibody diluted 50 fold. Anti-ICP27 and anti-UL42 antibodies were kindly provided by Dr Marsden (antibodies 42 and antibodies Z1F11 respectively) (Schenk *et al.,* 1988; Sinclair *et al.,* 1994). The corresponding proteins were then revealed by the ECL Western blotting analysis system (Amersham Pharmacia Biotech) using an anti-rabbit or an anti-mouse peroxydase-conjugate (Sigma) diluted 1:10000.

### Distribution of mRNAs among polyribosomal fractions.

Polyribosomes were prepared from about 8x10⁶ of mock-infected and infected cells. Fractionation of polyribosomes on sucrose gradients was carried out from post-mitochondrial supernatants as described in Greco *et al*. (1997). Twenty fractions of identical volume were collected from the top of the gradients. Each fraction was treated with 100 µg/ml proteinase K, and 1% SDS, for 15 min before phenol-chloroforme extraction and ethanol precipitation of the RNA. RNA from each fraction was resuspended in 10 mM TRIS-HCl, pH 7.4, 1 mM EDTA, 1 U of RNAse inhibitor (Pharmacia Biotec). The quantitative distribution of the various mRNAs among the different fractions was then analyzed by dot blots as described above.

### Antiviral susceptibility testing.

Three HSV 1 reference strains were tested : *SC16* (acyclovir-susceptible reference strain ; (Hill *et al.,* 1975)), *DM21* (acyclovir-resistant reference strain derived from *SC16* ; (Efstathiou *et al*., 1989)) and *TP* 2.5 (foscarnet-resistant reference strain derived from *SC16* ; (Larder and Darby, 1985)). Antiviral assays were performed in duplicate on HEp-2 cells seeded in 96 wells microplates. Dilutions of virus and drugs were prepared in E-MEM without FCS. Cells were first treated for 24 hours with MGBG using a series of 6 twofold serial dilutions from 12.5 to 400 µM. Infection was then achieved with 5 tenfold dilutions of cell-associated virus (10⁻¹ to 10⁻⁵). Each dilution was incubated with a series of 6 concentrations of each antiviral drug (from 12.5 to 400 µM in twofold dilutions for MGBG, from 0.16 to 500 µM in fivefold dilutions for acyclovir and from 31 to 1000 µM in twofold dilutions for foscarnet). Cycloheximide was used at 1.5 µg/ml to completely inhibit viral replication, and a control of virus titre with no antiviral drug was also included. After incubation at 36°C with 5% CO₂ for 24 hours post-infection, medium was replaced with freshly prepared dilutions of MGBG. Viral multiplication was checked 24 hours afterwards by an ELISA (Morfin *et al*., 1999), using a rabbit monoclonal anti-HSV 1 antibody (BO 114, Dako) and a protein A peroxidase-conjugated (BioRad). The substrate, 2,2'-azino-di(3-ethyl-benzthiazoline) sulfonic acid (ABTS) diluted in ABTS buffer (Boehringer, Mannheim), was added to each well. After 30 min at room temperature, the plates were briefly agitated and optical densities were read at 405 nm with a multichannel spectrophotomoter (Titertek-Multiskan). Optical density values were used with the Biolise program (Life Sciences International) to calculate the concentration of the drug causing a 50% inhibition of viral replication (IC50) by logistic regression analysis.

### EXAMPLE 2 : Inactivation of S-adenosyl methionine decarboxylase but not of ornithine decarboxylase inhibit HSV infection

It was previously shown that the synthesis of an unexpected high proportion of cellular proteins is sustained or stimulated during HSV-1 infection. They include ribosomal proteins and 28 proteins with p/ ranging from 4 to 7 (Greco *et al.,* 2000; Greco *et al*., 1997; Simonin *et al*., 1997). The identification of some of the later 28 proteins was undertaken in order to check their involvement in HSV-1 infection.

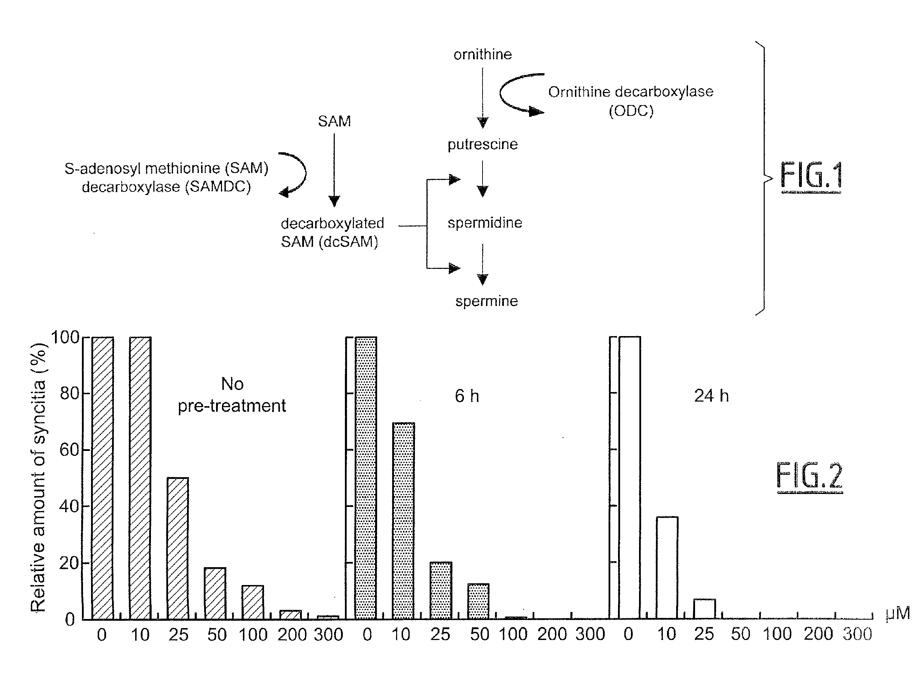

By western blotting of 2-D gels from proteins of HEp2 cells, one of the 28 cellular proteins which escape the virally-induced shutoff of cellular protein synthesis was identified as being the ornithine decarboxylase (ODC). This enzyme corresponds to protein number 198 described in (Greco *et al.,* 2000). In cells infected with HSV-1 for 3 hours and 6 hours, the ODC synthesis rate was respectively 4 fold and 1.2 fold higher than that in mock-infected cells (Greco *et al.,* 2000). ODC is involved in one of the first steps of the metabolic pathway of polyamines, in decarboxylating ornithine into putrescine (Figure 1). Spermine and spermidine are components of the HSV-1 viral particles; and localize respectively in the nucleocapsid and the envelope (Gibson and Roizman, 1971).

Accordingly, the effect of inactivation of enzymes involved in polyamine biosynthesis upon HSV-1 infection inhibition was assessed. The HSV-1 MP strain was used throughout this study for its ability to induce the formation of large syncytia from infected cells. Therefore, it was possible to easily and quickly investigate the inhibition of viral replication at the level of syncytia formation.

Difluoro methyl ornithine (DFMO) was used to inactivate ODC and consequently the synthesis of putrescine during the course of infection. HEp2 cells were treated for 24 h before infection with 1 to 10 mM DFMO, then submitted to HSV-1 infection in the presence of the same concentration of DFMO. In cells infected in the presence of DFMO the number of syncytia was not significantly reduced compared to cells infected in the absence of DFMO.

The role of another key enzyme of the polyamine pathway in HSV-1 replication was then investigated. The S-adenosyl methionine decarboxylase (SAMDC) acts downstream ODC. It decarboxylates S-adenosyl methionine (SAM) into decarboxylated SAM (dcSAM) which is a substrate in both spermidine and spermine biosynthesis. When HEp2 cells were infected in the presence of methylglyoxal bis(guanylhydrazone) (MGBG) which specifically inactivates SAMDC, HSV-1 infection was inhibited, even when MGBG was used at micromolar concentration. Therefore, the antiviral property of MGBG was further investigated by measuring its effect on viral infection at the level of syncytia formation, viral proteins and RNA accumulation, and viral DNA replication.

### EXAMPLE 3 : Quantitation of SAMDC expression in the course of HSV infection

### SAMDC mRNAs level during the course of HSV-1 infection

In order to determine whether SAMDC is submitted or not to HSV-1 induced shutoff of cellular protein synthesis during the course of infection, the amount of SAMDC mRNAs present in cells was estimated at different times after infection, together with that of β actin mRNA as a control of the viral induced shutoff of cellular protein synthesis. Total cytoplasmic RNAs were purified from cells either mock-infected, or infected for 6 h, 9 h, 12 h, and 24 h, and quantified by dot blot analysis using specific ³²P-labelled DNA probe. Results show that the amount of SAMDC mRNA increased progressively until the late stage of the viral infection (9 hpi).

### Efficiency of translation of SAMDC mRNA during the course of infection

To investigate whether SAMDC mRNA was efficiently translated during the course of infection, its behaviour among ribosomes and polyribosomes throughout infection was analyzed. To this aim, post-mitochondrial supernatants of cells, either mock-infected or infected during 6 h, 9 h, and 12 h were separated in twenty fractions after sucrose gradient centrifugation. The distribution of SAMDC mRNAs among the different fractions was then assessed by dot blot using specific ³²P-labelled DNA probe, and quantified. Results indicate that the distribution of SAMDC mRNAs varied according to the time of infection. Free mRNAs and mRNAs present onto the 40S ribosomal subunits progressively decreased during the course of infection, whereas that present onto polyribosomes containing 2 to 5 ribosomes per mRNA molecule progressively increased. These results suggest that SAMDC mRNAs are more efficiently translated after infection than before due to the initiation step which becomes more efficient.

All together, these experiments show that there is more SAMDC mRNA in HSV infected cells than in mock-infected cells and that SAMDC mRNAs are more efficiently translated after infection than before. This suggests that SAMDC synthesis is sustained during HSV-1 infection.

### EXAMPLE 4 : Alteration of the level of intracellular spermine in mock-infected and in HSV infected cells exposed to MGBG.

MGBG is known to alter the polyamine synthesis in inactivating SAMDC. Therefore, the amount of intracellular spermine was assessed in mock-infected and infected cells after MGBG exposure. Cells were grown for 12 h and 24 h in the presence of 100 µM MGBG which was added to cell medium simultaneously with virus particles for HSV-1 infected cells. Intracellular spermine content was estimated by standard procedure (Besson *et al.,* 1986; Birnbaum *et al.,* 1988; Seiler, 1970).

**Table 1 :**

| MGBG | Relative amount of intracellular spermine | | |
|---|---|---|---|
| treatment (100 µM) | Mock infected | Mock infected + MGBG | HSV infected + MGBG |
| 12 h | 100 ± 12,4 % | 60,6 ± 1,4 % | 61,8 ± 9,0 % |
| 24 h | 100 ± 7,3 % | 65,3 ± 14,3 % | 66,2 ± 7,0 % |
| means ± SE of data from 3 experiments | | | |

Results in table 1 show that data corresponding to 12 h and 24 h treatment were not significantly different. In the presence of MGBG there was a 35-40 % decrease in the amount of spermine in both mock-infected and HSV-1-infected cells. Indeed, the amount of intracellular spermine corresponded to about 60-65 % of that present in the control mock-infected cells grown in the absence of MGBG.

### EXAMPLE 5 : MGBG-induced inhibition of HSV infection

### Dose dependant inhibition of HSV-1 infection by MGBG

To investigate the effect of the concentration of MGBG on viral infection, HEp2 cells were infected with 0.5 PFU of HSV-1 per cell in FCS free medium in which 10 µM to 300 µM MGBG was added from the adsorption step of the virus until the end of the experiment. After 24 h of infection, the formation of syncytia was observed, and the number of syncytia was estimated by counting using the optical microscope. Results show that when 10 µM of MGBG was added to cell medium simultaneously with virus suspension, the size but not the number of syncytia was reduced. This indicates that viral multiplication was less efficient in the presence than in the absence of MGBG in the medium culture cell. At higher concentrations of MGBG, both the size and the number of syncytia were drastically decreased. There was 50 %, 95 % and about 99 % less syncytia formed in the presence of 50µM, 100µM and 200µM MGBG than in the control of cells infected in the absence of MGBG (left of figure 2). Therefore, MGBG precludes viral infection in a dose dependant manner. Depletion of cellular polyamines by addition of MGBG in cell medium together with viral suspension prevented the viral infection.

The same experiments were performed with cells grown in the presence of 5% FCS and different concentrations of MGBG. In these conditions, MGBG still inhibited viral infection and again the effect was dose dependant. However the inhibition was significantly less efficient than when cells were grown in a FCS free medium.

### More drastic antiviral MGBG effects are observed when cells are pre-exposed to MGBG before infection.

Since inhibition of viral infection by MGBG was more efficient in absence than in presence of FCS, we investigated the ability of MGBG to prevent HSV infection from cells pre-exposed to MGBG prior infection. HEp2 cell were pre-treated with MGBG by incubation in FCS free medium containing 10 to 300 µM MGBG 6h to 24 h before infection. Then cells were infected as described above and MGBG was maintained in the medium until the end of the experiment.

The formation of syncytia was shown to be less efficient in cells pre-exposed to MGBG prior infection than in non pre-treated cells. In addition, the efficiency of viral inhibition was depending on the time of pre-treatment. Histograms presented in figure 2 revealed that when cells were pre-treated with MGBG the inhibition of viral infection was very efficient even when the concentration of MGBG was low. Indeed, there was only 70% and 36% syncytia formed respectively in cells pre-treated for 6 h and 24 h with 10 µM MGBG compared to cells infected in the absence of MGBG. In cells pre-treated for 6 and 24 h with at least 25 µM MGBG, there was more than 80 % inhibition of viral infection. Therefore, the efficiency of inhibition of HSV-1 infection depended both on the concentration of MGBG and on the time of exposure of the cells to MGBG.

### HSV replication is inhibited even when MGBG is added into the cell after the penetration step of the virus

Entry of HSV into the host cell involves multistep process, including adsorption and penetration. To define the precise mechanism of blocking the viral replicative cycle by MGBG, the inhibition of viral replication was further examined when added either before or after the adsorption of the virus to HEp2 cells.

100 µM MGBG was added to cell medium either simultaneously with the viral particles or 2h, 4h and 6h after the beginning of the infection which corresponds to immediate-early, early and the end of the early phase of the viral cycle. When MGBG was added 2 h after the beginning of infection, the viral replication was totally inhibited. When MGBG was added 4 h or 6 h after the adsorption step of the virus, the viral replication was partially inhibited. Indeed, some syncytia were detected, indicating that viral replication occurred. However, the number of syncytia present in cells exposed to MGBG treatment from 4 and 6 h p.i. remained very low compared to that present in control cells infected in the absence of MGBG. Therefore, MGBG inhibited viral replication even when added after the penetration of the virus and when the viral replicative cycle had already started.

These results suggest that MGBG does not prevent the adsorption and the penetration of the virus to the cells.

### EXAMPLE 6 : At low concentrations MGBG does not inhibit cell growth and viability

The effect of MGBG on cell growth and viability was investigated. HEp2 cells were incubated in FCS free medium, in the presence of 10 µM to 500 µM of MGBG for 24 h, 30 h and 48 h. After harvesting, total cells, living cells and dead cells were counted.

There was no drastic difference in the growth rate of cells incubated for 24 h and 30 h in the presence of less than 200 µM MGBG compared to control cells grown in the absence of MGBG. However, cells incubated in the presence of 500 µM MGBG did not grow anymore. In addition, cell viability was not significantly reduced in cells treated with less than 500 µM MGBG for 24h and 30 h and with less than 25 µM MGBG for 48 h. Indeed, in these conditions, the percentage of dead cells did not exceed 5 % of total cells, whereas it reached 9 % and 24 % when cells were treated with 500 µM of MGBG for 24 h and 30 h respectively, and 10 % when cells were treated with 25 µM of MGBG for 48 h.

These results suggested that cell growth and cell viability were not affected when cells were submitted to 24 and 30 h incubation in the presence of 10 µM to 200 µM MGBG. For longer exposure time, both of them were slightly decreased in the presence of 25 µM to 200 µM MGBG. When cells were incubated in the presence of 500 µM MGBG, cell growth was altered even after 24 h exposure to MGBG, whereas cell viability was decreased only after 48 h exposure time.

### EXAMPLE 7 : addition of spermine reverses MGBG inhibition of HSV replication

### Addition of exogenous spermine prevents MGBG inhibition of viral replication.

The question arises whether inhibition of HSV-1 infection by MGBG was due to the depletion of polyamines intracellular content, or/and to the deficiency of *de novo* polyamine synthesis in MGBG treated cells. HEp2 cells were infected in the presence of MGBG alone or in combination with spermine. In these experiments, 100 µM of MGBG was added simultaneously with viral suspension. In the presence of MGBG alone, the viral infection was strongly reduced compared to cells infected without MGBG. On the contrary, the inhibition of viral infection was totally prevented when 50 µM spermine was added to cell medium simultaneously with MGBG and viral suspension, or when spermine was added 2 h later. Therefore, spermine given together with MGBG or 2 h later prevented the effect of MGBG.

### The levels of HSV-1 proteins, mRNA and DNA are decreased in cells infected in the presence of MGBG

To elucidate the mechanisms responsible for the inhibition of viral replication in polyamine-deficient HEp2 cells, the effect of MGBG in this process was investigated at the molecular level. We determined the amount of viral proteins, mRNA and DNA in cells infected in the absence and in the presence of MGBG.

Cells were pre-treated with 10 to 200 µM of MGBG, then infected with HSV-1 in the presence of the same concentrations of MGBG. Proteins, RNA and DNA were extracted from cells infected for 24 h. Viral proteins were detected by Western blot using specific antibodies, whereas viral mRNA and DNA levels were assayed by slot blot analysis.

Results show that depletion of cellular polyamines by treatment of cells with MGBG not only significantly decreased viral DNA level, but also that of viral mRNA and proteins. Furthermore, the decrease in the level of viral products is MGBG dose dependant.

### Spermine restores viral proteins, mRNA and DNA levels in cells infected in the presence of MGBG

The same experiments were performed in the presence of MGBG alone and MGBG plus spermine. Cells were grown for 6 h in medium containing 100 µM MGBG, then cells were infected with HSV-1 in the presence of MGBG alone or MGBG and 50 µM spermine. Viral replication was assessed at 24 h p.i.

Results show that viral replication was totally inhibited in the presence of MGBG alone. On the contrary, the presence of exogenous spermine abolished the MGBG-induced viral inhibition. The levels of viral proteins, mRNA and DNA were decreased in MGBG treated cells, and were restored when exogenous spermine was added at the very early time of infection. Therefore, the inhibition of virus infection by MGBG is reversed by addition of exogenous spermine.

### EXAMPLE 8 : MGBG inhibits the replication of acyclovir and of foscarnet resistant HSV-1 strains

The capacity of MGBG to preclude infection of three HSV-1 acyclovir and/or foscarnet resistant strains was tested. The reference strains used in this study were the *SC16* acyclovir-susceptible reference strain, the *DM21* acyclovir-resistant reference strain derived from *SC16* and the *TP* 2.5 foscarnet-resistant reference strain derived from SC16 (Efstathiou *et al.,* 1989; Hill *et al.,* 1975; Larder and Darby, 1985). In each experiment, dose response curves were constructed for actinomycin D which inhibits transcription, for acyclovir, and for MGBG. The ED values were determined. Susceptibility to MGBG, acyclovir and foscarnet of the HSV reference strains are presented in table 2.

**Table 2 :**

| Viral strain | Antiviral drug | | |
|---|---|---|---|
| | MGBG | Acyclovir | Foscarnet |
| Acyclovir-susceptible reference strain *SC16* | 103 [68 - 152] | 28 [20 - 37] | 224 [174 - 279] |
| Acyclovir-resistant reference strain *DM21* | 75 [48 - 131] | > 500 | 148 [125 - 295] |
| Foscarnet-resistant reference strain *TP2.5* | 82 [63 - 134] | 215 [198 - 247] | > 1000 |
| Data correspond to the median and the extremes obtained from at least three independent assays. | | | |

The strains presented the same susceptibility to MGBG, even though the virus was resistant to acyclovir, such as *DM21* or resistant to both acyclovir and foscarnet, such as *TP2.5*. Strains resistant to one or more antiviral drugs became some-what more susceptible to other antiviral drugs. Acyclovir-resistant strains exhibited higher susceptibility to foscarnet than acyclovir-susceptible strain (34% decreased IC50). In addition, MGBG revealed to be more active on the two strains resistant to acyclovir and/or foscarnet, with a 20 to 27% decreased IC50 compared to the value observed for the susceptible strain *SC16*.

In addition, MGBG inhibited replication of HSV-1 clinical acyclovir or foscarnet resistant isolates.

### MGBG potentiates the acyclovir-induced inhibition of HSV-1 infection

Acyclovir (ACV) is one of the most used conventional anti-HSV-1 drug. It is a nucleoside analogue which specifically inhibits the activity of two viral enzymes, the DNA polymerase and the thymidine kinase. MGBG was shown to inhibit *in vitro* the replication of acyclovir resistant HSV-1 strains. The anti-HSV-1 activity of MGBG and ACV was further compared. HEp2 cells were infected with 0.5 PFU of HSV-1 per cell in the presence of ACV and MGBG alone or in combination, and the antiviral effects were evaluated at the level of syncytia formation. The inhibition of viral infection is more drastic when the drugs are used in combination rather than alone. Therefore, MGBG potentiates the antiviral effect of ACV.

In conclusion, the above results demonstrate that MGBG inhibits viral infection more efficiently when cells are pre-treated with MGBG, *i.e.* when cells are depleted in polyamines. Nonetheless, MGBG inhibits viral replication even when added after the viral particles have penetrated into the cells. However, the inhibition is more efficient when added at a time when early viral events have not yet started. Spermine prevents viral inhibition even when added after the addition of MGBG. All together, these results suggest that MGBG does not inhibit viral entry into HEp2 cells but rather that inhibition of viral replication is mediated at least partially by the final product of the polyamines pathway.

In addition molecules derived from MGBG might represent useful additional drugs for antiviral therapy of HSV infections, especially in combination with acyclovir.

### REFERENCES

Akli S, Caillaud C, Vigne E, Stratford-Perricaudet LD, Poenaru L, Perricaudet M, Kahn A, Peschanski MR. (1993) Transfer of a foreign gene into the brain using adenovirus vectors. Nature Genetics, 3, 224-228.

Barbas CF, Bain JD, Hoekstra DM, Lerner RA. (1992), Semisynthetic combinatorial antibody libraries: a chemical solution to the diversity problem. PNAS USA, 89, 4457-4461.

Besson, M., Delbecque, J., Mathelin, J., Boisson, A. and Delachambre, J. (1986) Epidermal polyamine levels related to cell cycle events during the metamorphosis of Tenebrio molitor L. (Insecta, Coleoptera): effect ofjuvenoid application. Comp. Biochem. Physiol., 83B, 589-593.

Birnbaum, M., Whelan, T.M. and Gilbert, L. (1988) Temporal alterations in polyamine content and ornithine decarboxylase activity during the larval-pupal development of Manduca sexta. Insect Biochem, 18, 853-859.

Cech, T.R. (1989) RNA as an enzyme. Biochem Int., 18, 7-14.

Chiocca EA, Choi BB, Cai WZ, DeLuca NA, Schaffer PA, DiFiglia M, Breakefield XO, Martuza RL. (1990) Transfer and expression of the lacZ gene in rat brain neurons mediated by herpes simplex virus mutants. New Biol., 2, 739-746.

Colas P, Cohen B, Jessen T, Grishina I, McCoy J, Brent R. (1996) Genetic selection of peptide aptamers that recognize and inhibit cyclin-dependent kinase 2. Nature, 380, 548-50.

Danos O, Mulligan RC. (1988) Safe and efficient generation of recombinant retroviruses with amphotropic and ecotropic host ranges. Proc Natl Acad Sci U S A , 85, 6460-4.

Diaz, J.-J., Duc Dodon, M., Schaerer-Uthurralt, N., Simonin, D., Kindbeiter, K., Gazzolo, L. and Madjar, J.-J. (1996) Post-transcriptional transactivation of human retroviral envelope glycoprotein expression by herpes simplex virus Us11 protein. Nature, 379, 273-277.

Diaz, J.-J., Simonin, D., Massé, T., Deviller, P., Kindbeiter, K., Denoroy, L. and Madjar, J.-J. (1993) The herpes simplex virus type 1 Us11 gene product is a phosphorylated protein found to be non-specifically associated with both ribosomal subunits. J Gen Virol, 74, 397-406.

Dobson AT, Margolis TP, Sedarati F, Stevens JG, Feldman LT. (1990) A latent, nonpathogenic HSV-1-derived vector stably expresses beta-galactosidase in mouse neurons. Neuron, 5, 353-360.

Efstathiou, S., Kemp, S., Darby, G. and Minson, A. (1989) The role of herpes simplex virus type 1 thymidine kinase in pathogenesis. J Gen Virol, 70, 869-879.

Felgner PL, Gadek TR, Holm M, Roman R, Chan HW, Wenz M, Northrop JP, Ringold GM, Danielsen M. (1987) Lipofection: a highly efficient, lipid-mediated DNA-transfection procedure. Proc Natl Acad Sci U S A, 84,7413-7.

Felgner PL, Ringold GM. (1989) Cationic liposome-mediated transfection. Nature, 337, 387-8.

Fraley R, Subramani S, Berg P, Papahadjopoulos D. (1980) Introduction of liposome-encapsulated SV40 DNA into cells. J Biol Chem, 255,10431-5.

Gibson, W. and Roizman, B. (1971) Compartmentalization of spermine and spermidine in the herpes simplex virion. Proc Natl Acad Sci U S A, 68, 2818-2821.

Goldin, A.L., Sandri-Goldin, R.M., Levine, M. and Glorioso, J.C. (1981) Cloning of herpes simplex virus type 1 sequences representing the whole genome. Journal of Virology, 38, 50-58.

Graham FL, Smiley J, Russell WC, Nairn R. (1977) Characteristics of a human cell line transformed by DNA from human adenovirus type 5. J. Gen. Virol., 36, 59-74.

Graham FL. (1984) Covalently closed circles of human adenovirus DNA are infectious. EMBO J., 12, 2917-22.

Greco, A., Bausch, N., Couté, Y. and Diaz, J.-J. (2000) Characterization by two-dimensional gel electrophoresis of host proteins whose synthesis is sustained or stimulated during the course of herpes simplex virus type 1 infection. Electrophoresis, 21, 2522-2530.

Greco, A., Laurent, A.M. and Madjar, J.-J. (1997) Repression of β-actin synthesis and persistence of ribosomal protein synthesis after infection of HeLa cells by herpes simplex virus type 1 are under translational control. Mol Gen Genet, 256, 320-327.

Harrison (2000) Medecine Interne.- 14 ed .- Mc Graw-Hill International, ISBN 2 7042 13380.

Harlow E. et al., Antibodies: A Laboratory Manual, Cold Spring Harbor Laboratory, New York, (1988).

Hill, T.J., Field, H.J. and Blyth, W.A. (1975) Acute and recurrent infection with herpes simplex virus in the mouse: a model for studying latency and recurrent disease. J Gen Virol, 28, 341-353.

Jayasena S.D. (1999) Aptamers: an emerging class of molecules that rival antibodies in diagnostics. Clin Chem., 45,1628-50.

Kaneda Y, Iwai K, Uchida T. (1989) Increased expression of DNA cointroduced with nuclear protein in adult rat liver. Science, 243, 375-8.

Kohler and Milstein (1975) Continuous cultures of fused cells secreting antibody of predefined specificity. Nature, 256, 495-7.

Laemmli, U.K. (1970) Cleavage of structural proteins during the assembly of the head of bacteriophage T4. Nature, 227, 680-685.

Larder, B.A. and Darby, G. (1985) Selection and characterisation of acyclovir-resistant herpes simplex virus type 1 mutants inducing altered DNA polymerase activities. Virology, 146, 262-271.

Le Gal La Salle G, Robert JJ, Berrard S, Ridoux V, Stratford-Perricaudet LD, Perricaudet M, Mallet J. (1993) An adenovirus vector for gene transfer into neurons and glia in the brain. Science, 259, 988-990.

Levrero M, Barban V, Manteca S, Ballay A, Balsamo C, Avantaggiati ML, Natoli G, Skellekens H, Tiollais P, Perricaudet M. (1991) Defective and nondefective adenovirus vectors for expressing foreign genes in vitro and in vivo. Gene, 101, 195-202.

McCormick, F.P. and Newton, A.A. (1975) Polyamine metabolism in cells infected with herpes simplex virus. J Gen Virol, 27, 25-33.

Morfin, F., Thouvenot, D., De Turenne-Tessier, M., Lina, B., Aymard, M. and Ooka, T. (1999) Phenotypic and genetic characterization of thymidine kinase from clinical strains of varicella-zoster virus resistant to acyclovir. Antimicrob Agents Chemother, 43, 2412-2416.

Miyanohara A, Johnson PA, Elam RL, Dai Y, Witztum JL, Verma IM, Friedmann T. (1992) Direct gene transfer to the liver with herpes simplex virus type 1 vectors: transient production of physiologically relevant levels of circulating factor IX. New Biol. 4, 238-246.

Pagano JS, McCutchan JH, Vaheri A. (1967) Factors influencing the enhancement of the infectivity of poliovirus ribonucleic acid by diethylaminoethyl-dextran. J Virol., 1, 891-7.

Pajunen A, Crozat A, Janne OA, Ihalainen R, Laitinen PH, Stanley B, Madhubala R, Pegg AE (1988) Structure and regulation of mammalian S-adenosylmethionine decarboxylase. J. Biol. Chem., 263,17040-9.

Pohjanpelto, P., Sekki, A., Hukkanen, V. and von Bonsdorff, C.H. (1988) Polyamine depletion of cells reduces the infectivity of herpes simplex virus but not the infectivity of Sindbis virus. Life Sci, 42, 2011-2018.

Roemer K, Friedmann T. (1992) Concepts and strategies for human gene therapy. Eur. J. Biochem., 208, 211-25.

Rubin-Carrez C. (2000). Les mimes peptidiques. Le technoscope, Biofutur, vol 199.

Sambrook, Fritsch & Maniatis, Molecular Cloning: A Laboratory Manual, Second Edition (1989) Cold Spring Harbor Laboratory Press, Cold Spring Harbor, New York.

Schenk, P., Pietschmann, S., Gelderblom, H., Pauli, G. and Ludwig, H. (1988) Monoclonal antibodies against herpes simplex virus type 1-infected nuclei defining and localizing the ICP8 protein, 65K DNA-binding protein and polypeptides of the ICP35 family. Journal of General Virology, 69, 99-111.

Seiler, N. (1970) Use of the dansyl reaction in biochemical analysis. Methods Biochem Anal, 18, 259-337.

Sharp, P.A. (2001) RNA interference - 2001. Genes Dev, 15:485-490.

Simonin, D., Diaz, J.-J., Massé, T. and Madjar, J.-J. (1997) Persistence of ribosomal protein synthesis after infection of HeLa cells by herpes simplex virus type 1. J Gen Virol, 78, 435-443.

Sinclair, M., McLauchlan, J., Mardsen, H. and Brown, S. (1994) Characterization of a herpes simplex virus type 1 deletion variant (1703) which under-produces Vmw63 during immediate early conditions of infection. J Gen Virol, 75, 1083-1089.

Stratford-Perricaudet LD, Levrero M, Chasse JF, Perricaudet M, Briand P. (1990) Evaluation of the transfer and expression in mice of an enzyme-encoding gene using a human adenovirus vector. Human Gene Therapy, 1, 241-56.

Tuerk C. and Gold L. (1990) Systematic evolution of ligands by exponential enrichment: RNA ligands to bacteriophage T4 DNA polymerase. Science, 3, 249, 505-10.

Tyms, A., Scamans, E. and Williamson, J. (1979) Polyamine metabolism in MRC5 cells infected with different herpesviruses. Biochem Biophys Res Commun, 86, 312-318.

Waterhouse P, Griffiths AD, Johnson KS, Winter G. (1993) Combinatorial infection and in vivo recombination: a strategy for making large phage antibody repertoires. Nucleic Acids Research, 21, 2265-2266.

Wilson JM, Grossman M, Wu CH, Chowdhury NR, Wu GY, Chowdhury JR. (1992) Hepatocyte-directed gene transfer in vivo leads to transient improvement of hypercholesterolemia in low density lipoprotein receptor-deficient rabbits. J. Biol. Chem., 267, 963-967.

Wu GY, Wu CH. (1988) Receptor-mediated gene delivery and expression in vivo. J. Biol. Chem., 263, 14621-14624.

Yotnda P, Onishi H, Heslop HE, Shayakhmetov D, Lieber A, Brenner M, Davis A. (2001) Efficient infection of primitive hematopoietic stem cells by modified adenovirus. Gene Ther., 8, 930-7.

## Claims

1. Use of an inhibitor of S-adenosyl methionine decarboxylase (SAMDC) for the manufacture of a medicament intended for the prevention or the treatment of a herpes simplex virus infection.

2. The use according to claim 1, wherein said herpes simplex virus is HSV-1 or HSV-2.

3. The use according claim 1 or 2, wherein the inhibitor is administered in association with another agent efficient against a herpes simplex virus infection.

4. The use according to claim 3, wherein said agent is acyclovir.

5. The use according to any of claims 1 to 3, wherein said herpes simplex virus is a HSV-1 strain resistant to acyclovir and/or foscarnet and/or their derivatives.

6. The use according to any of preceding claims, wherein said medicament is intended for an administration to a non-human mammal.

7. The use according to any of claims 1 to 5, wherein said medicament is intended for an administration to a human.

8. The use according to claim 7, wherein said medicament is intended for an administration to an immunodepressed subject.

9. The use according to any of preceding claims, wherein said inhibitor is an inhibitor of SAMDC activity.

10. The use according to claim 9, wherein said inhibitor is methylglyoxal bis(amidinohydrazone).

11. The use according to any of claims 1 to 9, wherein said inhibitor is an inhibitor of SAMDC expression.

12. The use according to claim 11, wherein said inhibitor is an antisense nucleic acid sequence that blocks expression of SAMDC.

13. A pharmaceutical composition comprising an inhibitor of S-adenosyl methionine decarboxylase and another agent efficient against a herpes simplex virus infection, in a pharmaceutically acceptable carrier.

14. The pharmaceutical composition according to claim 13, wherein said agent is acyclovir.

15. The pharmaceutical composition according to claim 13 or 14, wherein said inhibitor is methylglyoxal bis(amidinohydrazone).


SEQUENCE LISTING

<110>  INSERM

<120>  S-adenosyl methionine decarboxylase inhibition for the treatment of a
herpes simplex virus infection

<130>  BET 02/0722

<160>  1

<170>  PatentIn version 3.1

<210>  1
<211>  1805
<212>  DNA
<213>  Homo sapiens

<400>  1
   aagagactga actgtatctg cctctatttc caaaagactc acgttcaact ttcgctcaca      60
   caaagccggg aaaattttat tagtcctttt tttaaaaaaa gttaatataa aattatagca     120
   aaaaaaaaaa ggaacctgaa ctttagtaac acagctggaa caatcgcagc ggcggcggca     180
   gcggcgggag aagaggttta atttagttga ttttctgtgg ttgttggttg ttcgctagtc     240
   tcacggtgat ggaagctgca cattttttcg aagggaccga gaagctgctg gaggtttggt     300
   tctcccggca gcagcccgac gcaaaccaag gatctgggga tcttcgcact atcccaagat     360
   ctgagtggga catacttttg aaggatgtgc aatgttcaat cataagtgtg acaaaaactg     420
   acaagcagga agcttatgta ctcagtgaga gtagcatgtt tgtctccaag agacgtttca     480
   ttttgaagac atgtggtacc accctcttgc tgaaagcact ggttcccctg ttgaagcttg     540
   ctagggatta cagtgggttt gactcaattc aaagcttctt ttattctcgt aagaatttca     600
   tgaagccttc tcaccaaggg tacccacacc ggaatttcca ggaagaaata gagtttctta     660
   atgcaatttt cccaaatgga gcaggatatt gtatgggacg tatgaattct gactgttggt     720
   acttatatac tctggatttc ccagagagtc gggtaatcag tcagccagat caaaccttgg     780
   aaattctgat gagtgagctt gacccagcag ttatggacca gttctacatg aaagatggtg     840
   ttactgcaaa ggatgtcact cgtgagagtg gaattcgtga cctgatacca ggttctgtca     900
   ttgatgccac aatgttcaat ccttgtgggt attcgatgaa tggaatgaaa tcggatggaa     960
   cttattggac tattcacatc actccagaac cagaattttc ttatgttagc tttgaaacaa    1020
   acttaagtca gacctcctat gatgacctga tcaggaaagt tgtagaagtc ttcaagccag    1080
   gaaaatttgt gaccaccttg tttgttaatc agagttctaa atgtcgcaca gtgcttgctt    1140
   cgccccagaa gattgaaggt tttaagcgtc ttgattgcca gagtgctatg ttcaatgatt    1200
   acaattttgt ttttaccagt tttgctaaga agcagcaaca acagcagagt tgattaagaa    1260
   aaatgaagaa aaaacgcaaa aagagaacac atgtagaagg tggtggatgc tttctagatg 1320
   tcgatgctgg gggcagtgct ttccataacc accactgtgt agttgcagaa agccctagat 1380
   gtaatgatag tgtaatcatt ttgaattgta tgcattatta tatcaaggag ttagatatct 1440
   tgcatgaatg ctctcttctg tgtttaggta ttctctgcca ctcttgctgt gaaattgaag 1500
   tggatgtaga aaaaaccttt tactatatga aactttacaa cacttgtgaa agcaactcaa 1560
   tttggtttat gcacagtgta atatttctcc aagtatcatc caaaattccc cacagacaag 1620
   gctttcgtcc tcattaggtg ttggcctcag cctaaccctc taggactgtt ctattaaatt 1680
   gctgccagaa ttttacatcc agttacctcc actttctaga acatattctt tactaatgtt 1740
   attgaaacca atttctactt catactgatg tttttggaaa cagcaattaa agtttttctt 1800
   ccatg 1805